# EUROPÄISCHE PATENTANMELDUNG

(11) **EP 1 386 911 A2**
(43) Veröffentlichungstag der Anmeldung: **04.02.2004**
(21) Anmeldenummer: 03016108.7
(22) Anmeldetag: 16.07.2003
(51) Int. Cl.: C07C 303/32

(54) **Verfahren zur Herstellung von Acyloxybenzolsulfonaten**

(30) Priorität: 25.07.2002 DE 10233827
(71) Anmelder: Clariant GmbH, 65929 Frankfurt (DE)
(72) Erfinder: Reinhart, Gerd, Dr., 65779 Kelkheim (DE); Naumann, Peter, Dr., 65232 Taunusstein (DE); Lerch, Alexander, Dr., 63571 Gelnhausen (DE); Mueller, Wolf-Dieter, Dr., Charlotte, NC 28277 (Mecklenburg County) (US); Sadanani, Narayan D., Dr., Charlotte, NC 28210 (Mecklenburg County) (US)
(74) Vertreter: Paczkowski, Marcus, Dr.

(57) **Zusammenfassung**

Verfahren zur Herstellung von Acyloxybenzolsulfonaten durch Umsetzung von wasserfreien Phenolsulfonaten mit Carbonsäurederivaten, wobei man die Umsetzung mit einem Salz einer Phenolsulfonsäure durchführt, das einen Wassergehalt von weniger als 0,5 Gew.-% aufweist, und das mit einer Substanz mit basischen Eigenschaften in Kontakt gebracht wurde.

## Beschreibung

Die Erfindung betrifft ein Verfahren zur Herstellung von Acyloxybenzolsulfonaten, ausgehend von Carbonsäurederivaten und wasserarmen Salzen einer Phenolsulfonsäure.

Acyloxybenzolsulfonsäuren und ihre Salze sind seit langem bekannte Verbindungen. In Abhängigkeit von der Kettenlänge der Acylgruppe können sie als Tenside, Bleichaktivatoren oder in anderen Anwendungen Verwendung finden.
DE 666 626 beschreibt ihre tensidischen Eigenschaften und ihre allgemeine Verwendung in Waschmitteln, während Verbindungen mit 6 bis 12 Kohlenstoffatomen in der Alkylkette in Kombination mit Persalzen durch EP 98 129, EP 105 672, EP 105 673 und EP 125 641 als Bleichmittel beansprucht werden.

Zur Herstellung von Acyloxybenzolsulfonsäuren und ihren Salzen sind eine Vielzahl von Methoden beschrieben. Sie können durch Erhitzen eines Gemisches aus Trifluoressigsäureanhydrid, Natriumphenolsulfonat (SPS) und einer (C₆-C₁₉) Alkancarbonsäure erhalten werden. Nach US 4,587,054 kann diese Reaktion auch zweistufige Reaktionsfolge durchgeführt werden: Zunächst wird die Alkancarbonsäure in Gegenwart eines Überschusses von Acetanhydrid in das Anhydrid umgewandelt, anschließend wird das isolierte Anhydrid mit trockenem Phenolsulfonat umgesetzt. Diese Reaktion erfolgt bei Temperaturen von 180 bis 220°C unter Basenkatalyse. Die säurekatalysierte Umsetzung eines längerkettigen Alkansäureanhydrids mit SPS in einem aprotischen Lösemittels ist in US 4,588,532 beansprucht, die saure Katalyse (Toluolsulfonsäure und verwandte Verbindungen) erlaubt eine Reaktionsführung schon bei 120°C.
Literaturbekannt ist weiterhin die Umesterung eines (C₂-C₃)Acyloxybenzolsulfonats mit einer (C₆-C₈)Alkancarbonsäure unter gleichzeitiger Entfernung der gebildeten kurzkettigen Alkancarbonsäure. Ebenso ist die Umsetzung von Alkalimetall- oder Erdalkalimetallphenolsulfonaten mit einem C₂-C₃₁-Alkanphenylesters bei 200 bis 350°C möglich.

Eine weitere Herstellungsvariante ist die Umsetzung von aliphatischen oder aromatischen Carbonsäurehalogeniden mit Salzen der Phenolsulfonsäure. Die Reaktion kann unter Schotten-Baumann-Bedingungen im wässrigen System durchgeführt werden (US 5,523,434), führt dann allerdings nur zu mäßigen Umsetzungsgraden. Vorteilhafter ist die Umsetzung von wasserfreien Salzen von Phenolsulfonsäuren in wasserfreien Medien. Als Reaktionsmedium dienen organische Lösungsmittel wie Methylenchlorid (US 35 03 888), hochsiedende Kohlenwasserstoffe (EP 220 826), Xylol oder Toluol (EP 164 786) und Trifluoressigsäure (WO 01/19 771). Gemäß US 5 069 828 wird diese Reaktion in einem aprotischen organischen Lösungsmittel in Anwesenheit eines Phasentransferkatalysators durchgeführt. Nach US Patent Anmeldung 20 020 058 824 kann diese Reaktion auch lösemittelfrei durchgeführt werden, wenn mit einem Überschuss an Säurechlorid gearbeitet wird.

Bei allen bekannten technisch verwertbaren Verfahren ergibt sich das Problem, dass für die Reaktion nahezu wasserfreies SPS benutzt werden muss, da andernfalls das Carbonsäurederivat (Halogenid oder Anhydrid) bzw. der fertige Ester in Gegenwart von Spuren von Wasser hydrolysieren, was zu erheblichen Ausbeuteverlusten führt. Nahezu wasserfrei meint in diesem Fall Wassergehalte < 0,5 Gew.-%, vorzugsweise < 0,2 Gew.-%.

SPS ist kommerziell als Dihydrat mit einem Wasseranteil von ca. 15 Gew.-% verfügbar. Durch konventionelle Trocknung kann der Wassergehalt auf ca. 2 Gew.-% gesenkt werden. Nach US 5,069,828 ist es möglich, die Restwassermenge durch azeotrope Destillation in Gegenwart eines Schleppmittels wie Xylol zu entfernen. Wegen des enormen Zeitbedarfs ist dies aber großtechnisch oder in kontinuierlich arbeitenden Anlagen wenig sinnvoll.

Wie aus US 4,666,636 bekannt ist, kann der Wassergehalt durch spezielle Trocknung in entsprechenden Apparaturen auf unter 0,5 Gew.-% gesenkt werden.

Hierzu müssen jedoch bestimmte Trocknungsbedingungen (170 bis 200°C, Inertgas, Vakuum, Fließbetttrockner, gute Durchmischung) exakt eingehalten werden. Werden diese Bedingungen nicht exakt eingehalten, geht SPS eine Reihe von Nebenreaktionen ein, wodurch das Produkt irreversibel geschädigt wird. Dies hat zur Folge, dass sowohl der Umsetzungsgrad der folgenden Acylierung, wie auch die Farbe des Endprodukte signifikant negativ beeinflusst wird. Falsche Trocknung, d.h. zu lange Verweilzeiten oder zu hohe Temperaturen führen zur Übertrocknung des SPS, was in der Acylierungsreaktion zu Umsetzungsgraden kleiner 50 % führt. Dies kann auch durch überstöchiometrischen Einsatz der Acylierungskomponente nicht verbessert werden. Im großtechnischen Betrieb der Trocknung von SPS sind diese spezifischen physikalischen Parameter jedoch nur schwer exakt einzuhalten. Hilfreich wäre es Wege zu finden, die störenden Nebenreaktionen während der Trocknung zu unterbinden, um die Produktqualität des SPS unabhängig von Trockenzeit, -temperatur und anderen physikalischen Parametern zu machen.

Aufgabe der vorliegenden Erfindung ist es deshalb, ein sowohl großtechnisch als auch kontinuierlich durchführbares Verfahren zu entwickeln, das eine optimale Trocknung des SPS, auch außerhalb der in US 4,666,636 genannten Reaktionsbedingungen erlaubt, ohne Verlust an Reaktivität des SPS in der folgenden Acylierungsstufe in Kauf nehmen zu. Dabei soll das Verfahren unabhängig von den physikalischen Trocknungsparametern des eingesetzten Natrium-Phenolsulfonats sein.

Überraschenderweise wurde nun gefunden, dass, ausgehend von wasserfreiem SPS, Acyloxybenzolsulfonate hergestellt werden können, unabhängig von der thermischen Vorbehandlung des eingesetzten SPS, wenn das wasserfreie SPS nach seiner Herstellung und Isolierung, aber vor der Umsetzung mit dem Carbonsäurederivat mit mindestens einer Substanze mit basischen Eigenschaften in Kontakt gebracht wird. Unabhängig von den physikalischen Trocknungsparametern reagiert dieses SPS dann mit Acylierungsagenzien in ausgezeichneten Ausbeuten zu Acyloxybenzolsulfonaten von hervorragender Qualität.

Gegenstand der Erfindung ist ein Verfahren zur Herstellung von Acyloxybenzolsulfonaten durch Umsetzung von wasserfreien Phenolsulfonaten mit Carbonsäurederivaten, dadurch gekennzeichnet, dass das Salz einer Phenolsulfonsäure nach seiner Isolierung, aber vor der Acylierung mit mindestens einer Substanz mit basischen Eigenschaften in Kontakt gebracht wird.

Bei den als Ausgangsverbindungen dienenden Phenolsulfonaten handelt es sich vorzugsweise um Verbindungen der Formel 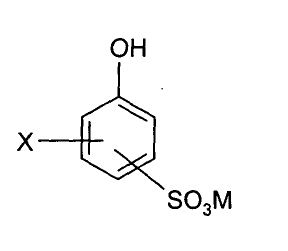 wobei X Wasserstoff, Halogen oder C₁-C₄-Alkyl und M ein Alkali- oder Erdalkalimetall-ion bedeutet. Bevorzugt sind Natrium-ortho- oder para-Phenolsulfonate, insbesondere Natrium-para-phenolsulfonat (SPS), das herstellbedingt isomere Nebenprodukte (bis 10 %) oder andere Verunreinigungen in geringen Mengen enthalten kann.

SPS wird hergestellt durch Sulfierung von Phenol und anschließende Neutralisation. Da Na-p-Phenolsulfonat in Wasser schwer löslich ist, kann es durch Filtration, Zentrifugation oder ähnliche Prozesse aus den Reaktionsmedium isoliert werden. Das Roh-SPS wird anschließend gewaschen und weist nach der Isolierung eine hohe Reinheit und einen Wassergehalt von 15 bis 30 % auf. Für die erfindungsgemäße Reaktion mit einem Carbonsäurederivat muss das Phenolsulfonat auf eine Restfeuchte von < 0,5, vorzugsweise < 0,2 Gew.-% getrocknet werden. Dieser Prozess kann kontinuierlich oder stufenweise über die Stufen des Dihydrats (Wassergehalt ca. 15 Gew.-%) und Viertelhydrats (Wassergehalt ca. 2 Gew.-%) erfolgen. Diese kann nach üblichen und an sich bekannten Verfahren geschehen, beispielsweise in einem Scheibentrockner oder Fließbetttrockner, der eine Trocknung auf eine Restfeuchte von weniger als 0,1 Gew.-% erlaubt. Während der Trocknung ist es vorteilhaft, unter ein Inertgasstrom zu arbeiten. Bei der Trocknung kann unter Vakuum oder mit gleichem Ergebnis auch ohne Vakuum gearbeitet werden.

Die Trockenzeiten können in Abhängigkeit von der verwendeten Ausrüstung zwischen 1 min und 18 h liegen, die Temperaturen zwischen 80 und 250°C. Bei dem erfindungsgemäßen Verfahren hat die thermische Vorbehandlung des getrockneten SPS keinen Einfluss auf die Ausbeute der Acylierungsreaktion und es können im Schnitt Umsätze größer 95 % erzielt werden. Insbesondere können auch Trockenbedingungen angewandt werden, die außerhalb der in US 4,666,636 genannten optimalen Trockenbedingungen liegen, d.h. solche, die zu "übertrocknetem" Produkt führen. Ein solches Produkt kann gemäß dem Stand der Technik, nicht für Acylierungsreaktionen verwendet werden, da es nicht reaktiv genug ist.

Als Substanzen mit basischen Eigenschaften kommen alle organischen oder anorganischen Verbindungen in Frage, die in wässriger Lösung unter Bildung von Hydroxylionen dissoziieren. Verwendet werden insbesondere anorganische Basen, wie Alkali- oder Erdalkalioxide, -hydroxide, -carbonate, -hydrogencarbonate, -phosphate etc. Besonders bevorzugt sind Natriumcarbonat, Natriumhydrogencarbonat und Natriumhydroxid, daneben aber auch die entsprechenden K-Salze.

Die Basen können sowohl wasserfrei, d.h. als Pulver, als Slurry, Paste oder als wässrige Lösung mit dem SPS in Kontakt gebracht werden. Dies kann direkt nach der Isolierung des SPS, d.h. nach dem Waschen des Filterkuchens oder vor bzw. während der Trocknung zum Dihydrat oder Viertelhydrat erfolgen. Alternativ kann das Inkontaktbringen auch noch während der anschließenden Trocknung zum wasserfreien SPS erfolgen. Die Zugabe kann sowohl in geeigneten Apparaturen, wie Mischern erfolgen oder auch direkt vor oder während der Trocknung selbst. In diesem speziellen Fall geschieht dies am vorteilhaftesten entweder durch Aufsprühen der gelösten Base direkt in die Trocknungsapparatur oder durch kontinuierliche Fütterung parallel zum feuchten SPS bei der Beschickung des Trockners.

Die Menge der benötigten Base liegt zwischen 0,01 und 10 Gew.-%, vorzugsweise 0,1 bis 5 Gew.-% bezogen auf das SPS in Form des Dihydrats (Wassergehalt ca. 15 Gew.-%).
Als Carbonsäurederivate können sowohl die Halogenide als auch die Anhydride der allgemeinen Formel

R-C(O)-X

mit X = Cl, Br, O-C(O)-R,
wobei R C₁-C₁₈ lineare oder verzweigte Alkylreste, wobei die Alkylgruppe ggf. durch eine Ester- oder Amidgruppe unterbrochen sein kann, oder
C₅-C₁₁ Arylreste sein können, die ggf. Heteroatome wie Stickstoff enthalten können und ggf. substituiert sind,
verwendet werden.

Als Carbonsäure können lineare oder verzweigte, gesättigte oder ungesättigte Alkancarbonsäuren mit 1 bis 22 Kohlenstoffatomen verwendet werden. Beispiele hierfür sind Essigsäure, Hexansäure, Heptansäure, Octansäure, Methyloctansäure, Nonansäure, 3,3,5-lsononansäure, Decansäure, Undecansäure, Undecensäure, Laurinsäure, Myristinsäure, gehärtete Talgfettsäure, Stearinsäure, Benzoesäure oder Chlorbenzoesäure wiedergegeben. Besonders bevorzugt werden Octansäure, Nonansäure, Isononansäure, Decansäure und Laurinsäure. Die Alkancarbonsäure kann weitere Substituenten wie Halogene, Nitro- oder Aminogruppen tragen oder durch Sauerstoffatome, Ester- bzw. Amidofunktionen unterbrochen sein. Beispiele hierfür sind n-Octylchlorameisensäure, Nonylchlorameisensäure, Octanoyloxyessigsäurechlorid, Phthalimidohexansäurechlorid und Nonanoylamidohexansäurechlorid.

Es kommen insbesondere die Carbonsäurechloride oder -bromide in Frage, wobei die Chloride bevorzugt sind. Diese können aus den entsprechenden Carbonsäuren z.B. durch Umsetzung mit Phosgen, Thionylchlorid, Phosphortrichlorid, Phosphoroxychlorid, Phosphorpentachlorid oder Phosphortribromid hergestellt werden.
Bei den Anhydriden können symmetrische oder unsymmetrische Verbindungen eingesetzt werden. Bespiele hierfür sind Acetanhydrid, Nonansäureanhydrid, lsononasäureanhydrid, Benzoesäureanhydrid, Octansäureanhydrid oder Acetylnonansäureanhydrid.
Carbonsäurederivat und Phenolsulfonat gemäß der Erfindung können vorzugsweise im Molverhältnis von 0,8 : 1 bis 2 : 1, vorzugsweise 1 : 1 bis 1,5 : 1 miteinander umgesetzt werden.

Die Acylierung kann in gängigen protischen oder aprotischen Lösungsmittel oder in einem Überschuss der entsprechenden Säure durchgeführt werden. Als Reaktionsmedium besonders bevorzugt werden aliphatische oder aromatische Kohlenwasserstoffe mit Siedepunkten zwischen 80 und 220°C, insbesondere 100 bis 180°C, z.B. Toluol, Xylol, Paraffine mit 8 bis 22 Kohlenstoffatomen, wie Decan, Undecan, Dodecan, Hexadecan oder Octadecan oder deren Mischungen.
Besonders geeignet sind aliphatische Kohlenwasserstoffgemische wie sie als Shellsole (Shell), ISOPAR G und ISOPAR 4 (ESSO) kommerziell verfügbar sind. Die Löslichkeit des SPS in diesem Reaktionsmedium liegt häufig unter 1 %.

Ein zusätzlicher Katalysator ist normalerweise nicht notwendig, kann jedoch in einzelnen Fällen Vorteile bieten. Bevorzugt sind offenkettige oder cyclische tertiäre Amine oder Carbonsäureamide (wie in DE 101 29 663.5 beschrieben), Phasentransferkatalysatoren oder saure Katalysatoren wie p-Toluolsulfonsäure. Das Molverhältnis des eingesetzten Katalysators zum Phenolsulfonat beträgt 0,0001:1 bis 0,02 : 1, vorzugsweise 0,005 : 1 bis 0,012 : 1.

Die Acylierungsreaktion wird bei Temperaturen zwischen 60 und 200°C, insbesondere zwischen 100 und 150°C durchgeführt. Das während der Reaktion entstehende Gas wird abgeleitet, ggf. wird die Reaktion mit einem inerten Gasstrom aus Stickstoff oder Argon überlagert. Die Reaktion wird im Sinne einer heterogenen Reaktion (Slurry) durchgeführt, da weder das Phenolsulfonat noch das entstehende Acyloxybenzolsulfonat eine nennenswerte Löslichkeit im Reaktionsmedium aufweisen. Die Reaktionszeit richtet sich nach den Reaktionsbedingungen und kann zwischen 10 min und 5 h, vorzugsweise 30 bis 120 min betragen.

In einer besonderen Ausführungsform kann die erfindungsgemäße Reaktion kontinuierlich durchgeführt werden. Hierzu besonders geeignet sind Kesselkaskaden bzw. Rohrreaktoren, wie sie dem Fachmann bekannt sind.

Nach beendeter Reaktion wird das Reaktionsprodukt mittels konventioneller Trennmethoden isoliert. Hierzu eignen sich Zentrifugen, Filterapparate. Zur völligen Abtrennung des Katalysators empfiehlt es sich, das feste Reaktionsprodukt ein oder mehrmals mit dem Reaktionsmedium auszuwaschen. Die Mutterlauge kann ohne weitere Reinigung für die nächsten Reaktionen verwendet bzw. cycliert werden. Das gebildete Acyloxybenzolsulfonat fällt in hohen Ausbeuten in Form eines weißen Pulvers an, das durch konventionelle Trocknung isoliert werden kann.

Das auf diese Weise gewonnene Acyloxybenzolsulfonat kann als Tensid oder Persalzaktivator in Wasch- und Reinigungsmittel wie pulverförmigen Vollwaschmitteln, Fleckensalzen oder pulverförmigen Maschinengeschirrspülmitteln eingesetzt werden: Zur Erhöhung der Lagerstabilität in diesen Formulierungen kann es, wie dem Fachmann bekannt, in eine granulare Form überführt werden.

### Beispiele

### Beispiel 1 (Vergleichsbeispiel):

### Herstellung von SPS Filterkuchen

Natriumparaphenolsulfonat-dihydrat wurde hergestellt durch Neutralisation einer Lösung von Phenolsulfonsäure. Das Produkt wurde durch Filtration isoliert und mit Wasser gewaschen. Man erhielt einen Filterkuchen (SPS Filterkuchen) bestehend aus weißen Kristallen mit folgender Zusammensetzung > 98 % p-Phenolsulfonat-Natrium, < 2 % o-Phenolsulfonat-Natrium, < 0,5 % 2,4-Disulfonat-Natrium sowie < 0,5 % Sulfone (ermittelt auf der Grundlage der wasserfreien Substanz).

### Beispiel 2

a) Herstellung von wasserfreiem SPS (gemäß US 4,666,636)
   Das Produkt wurde bei 120°C getrocknet und wies anschließend einen Wassergehalt von 2 bis 2,5 % auf (Viertelhydrat). Anschließend wurde das restliche Wasser gemäß US 4,666,636, Beispiel 4 (30 Min., 180°C) entfernt. Man erhielt SPS mit einem Wassergehalt von ca. 0,2 %.
b) Verwendung von wasserfreiem SPS
   Synthese von Nonanoyloxybenzolsulfonat-Natrium
   98,1 g (0,5 mol) getrocknetes Phenolsulfonat-Natrium, hergestellt nach Beispiel 2a) wurden in 150 g ISOPAR G vorgelegt und auf 120°C erwärmt. Anschließend wurden 114,8 g (0,65 mol) Nonansäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 - 130°C über Nacht getrocknet.
   Telquel-Ausbeute: 164,9 g (Ausbeute 98 %) weißen Pulvers mit einem Nonanoyloxybenzolsulfonat-Natrium (NOBS) Gehalt von 98,5 %. Ausbeute
   NOBS: 96 %

### Beispiel 3 (Vergleichsbeispiel)

a) Herstellung von wasserfreiem SPS (außerhalb US 4,666,636)
   SPS Filterkuchen wurde analog Beispiel 1 hergestellt und anschließend bei 120°C zum Viertelhydrat getrocknet. Das Produkt wurde anschließend 12 h lang bei 180°C getrocknet und hatte einen Wassergehalt von 0,1 %.
b) Verwendung von wasserfreiem SPS
   Synthese von Nonanoyloxybenzolsulfonat-Natrium
   98,1 g (0,5 mol) getrocknetes Phenolsulfonat-Natrium, hergestellt nach Beispiel 3a) wurden in 150 g ISOPAR G vorgelegt und auf 120°C erwärmt. Anschließend wurden 114,8 g (0,65 mol) Nonansäurechlorid innerhalb von 30 min zugetropft und bei 120°C nachgerührt. Das dabei entstehende HCI-Gas wurde abgeleitet. Das Reaktionsgemisch wurde nach 2 h auf 80°C abgekühlt und über einen Filter abfiltriert. Das graue Reaktionsprodukt wurde 2 mal mit wenig ISOPAR G nachgewaschen und anschließend im Trockenschrank bei 110 bis 130°C über Nacht getrocknet.
   Telquel-Ausbeute: 129,0 g (Ausbeute 77 %) eines beige-braunen Pulvers mit einem Nonanoyloxybenzolsulfonat-Natrium (NOBS) Gehalt von 58 %. Ausbeute
   NOBS: 44 %.

### Beispiel 4:

a) Herstellung von wasserfreiem SPS
   SPS Filterkuchen wurde analog Beispiel 1 hergestellt, anschließend mit 0,05 N Natriumhydrogencarbonat-Lösung gewaschen und anschließend bei 120°C zum Viertelhydrat getrocknet. Das Restwasser wurde anschließend 12 h lang bei 180°C getrocknet und hatte anschließend einen Wassergehalt von 0,2 %.
b) Verwendung von wasserfreiem SPS
   Synthese von Nonanoyloxybenzolsulfonat-Natrium
   98,1 g (0,5 mol) getrocknetes Phenolsulfonat-Natrium, hergestellt nach Beispiel 4a) wurden in 150 g ISOPAR G vorgelegt und auf 120°C erwärmt. Anschließend wurden 114,8 g (0,65 mol) Nonansäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 bis 130°C über Nacht getrocknet.
   Telquel-Ausbeute: 164,8 g (Ausbeute 99 %) weißen Pulvers mit einem Nonanoyloxybenzolsulfonat-Natrium (NOBS) Gehalt von 98 %. Ausbeute
   NOBS: 97 %

### Beispiel 5

a) Herstellung von wasserfreiem SPS
   p-Phenolsulfonat-Natrium Filterkuchen wurde analog Beispiel 1 hergestellt, mit 5 % Natriumcarbonat-Lösung auf pH 7 eingestellt und anschließend bei 120°C zum Viertelhydrat getrocknet. Das Restwasser wurde anschließend 12 h lang bei 180°C getrocknet und hatte anschließend einen Wassergehalt von 0,15 %.
b) Verwendung von wasserfreiem SPS
   Synthese von Nonanoyloxybenzolsulfonat-Natrium
   Die Acylierung des nach Beispiel 5 a) hergestellten SPS mit Nonansäurechlorid erfolgt gemäß Beispiel 4b)
   Telquel-Ausbeute: 164,8 g (Ausbeute 98 %) weißen Pulvers mit einem Nonanoyloxybenzolsulfonat-Natrium (NOBS) Gehalt von 98 %. Ausbeute
   NOBS: 96 %

### Beispiel 6

a) Herstellung von wasserfreiem SPS
   p-Phenolsulfonat-Natrium Filterkuchen wurde analog Beispiel 1 hergestellt, mit 125 ml einer auf pH 6,9 eingestellten 0,2 N Natriumdihydrogenphosphat Lösung gewaschen und anschließend bei 120°C zum Viertelhydrat getrocknet. Das Restwasser wurde anschließend 12 h lang bei 180°C getrocknet und hatte anschließend einen Wassergehalt von 0,25 %.
b) Verwendung von wasserfreiem SPS
   Synthese von Nonanoyloxybenzolsulfonat-Natrium
   Die Acylierung des nach Beispiel 6 a) hergestellten SPS mit Nonansäurechlorid erfolgt gemäß Beispiel 4b)
   Telquel-Ausbeute: 162,1 g (Ausbeute 96 %) weißen Pulvers mit einem Nonanoyloxybenzolsulfonat-Natrium (NOBS) Gehalt von 98 %. Ausbeute
   NOBS: 94 %

### Beispiel 7

a) Herstellung von wasserfreiem SPS
   p-Phenolsulfonat-Natrium Filterkuchen wurde analog Beispiel 1 hergestellt und anschließend bei 120°C zum Viertelhydrat getrocknet. Anschließend wurden 2 % einer 10 %igen NaHCO₃ Lösung aufgesprüht und anschließend das Restwasser innerhalb von 12 h lang bei 180°C entfernt. Das Produkt hatte anschließend einen Wassergehalt von 0,18 %.
b) Verwendung von wasserfreiem SPS
   Synthese von Lauroyloxybenzolsulfonat-Natrium
   98,1 g (0,5 mol) getrocknetes Phenolsulfonat-Natrium, hergestellt nach Beispiel 7a) wurden in 150 g ISOPAR G vorgelegt und auf 120°C erwärmt. Anschließend wurden 142 g (0,65 mol) Laurinsäurechlorid innerhalb von 30 min zugetropft und bei 130°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 bis 130°C über Nacht getrocknet.
   Telquel-Ausbeute: 185,5 g (Ausbeute 98 %) eines weißen Pulvers mit einem Lauroyloxybenzolsulfonat-Natrium (LOBS) Gehalt von 98 %. Ausbeute
   LOBS: 96 %.

### Beispiel 8

a) Herstellung von wasserfreiem SPS
   p-Phenolsulfonat-Natrium Filterkuchen wurde analog Beispiel 1 hergestellt und anschließend bei 120°C zum Viertelhydrat getrocknet. Das Viertelhydrat wurde anschließend mit 2 % fein gemahlenem NaHCO₃ gemischt und dann das Restwasser 12 h lang bei 180°C getrocknet. Das SPS hatte anschließend einen Wassergehalt von 0,15 %.
b) Verwendung von wasserfreiem SPS
   Herstellung von 2-Methyloctanoyloxybenzolsulfonat-Natrium
   98,1 g (0,5 mol) getrocknetes Phenolsulfonat-Natrium, hergestellt nach Beispiel 8a) wurden in 150 g ISOPAR G vorgelegt und auf 120°C erwärmt. Anschließend wurden 114,7 g (0,65 mol) 2-Methyloctansäurechlorid innerhalb von 30 min zugetropft und bei 120°C nachgerührt. Das dabei entstehende HCI-Gas wurde abgeleitet. Das Reaktionsgemisch wurde nach 2 h auf 80°C abgekühlt und über einen Filter abfiltriert. Das weiße Reaktionsprodukt wurde 2 mal mit wenig ISOPAR G nachgewaschen und anschließend im Trockenschrank bei 110 bis 130°C über Nacht getrocknet.
   Telquel-Ausbeute: 160,9 g (Ausbeute 95,7 %) eines weißen Pulvers mit einem 2-Methyloctanoyloxybenzolsulfonat-Natrium Gehalt von 95 %.

### Beispiel 9

a) Herstellung von wasserfreiem SPS
   p-Phenolsulfonat-Natrium Filterkuchen wurde analog Beispiel 1 hergestellt und anschließend 1,5 % 0,1 N NaHCO₃ Lösung hinzugegeben, dann bei 120°C zum Viertelhydrat getrocknet. Das Restwasser wurde anschließend 12 h lang bei 180°C getrocknet und hatte anschließend einen Wassergehalt von 0,2 %.
b) Verwendung von wasserfreiem SPS
   Herstellung von Nonanoylamidocaproyloxybenzolsulfonsäure Natriumsalz 135,7 g (0,5 mol) n-Nonanoylamidohexansäure wurden bei 90°C aufgeschmolzen, 59,5 g (0,5 mol) Thionylchlorid wurden im Laufe von 2 h hinzugetropft: Das Reaktionsgemisch wurde 1h nachgerührt und entgast. Das resultierende n-Nonanoylamidohexansäurechlorid und 100,1 g (0,5 mol) wasserfreies SPS gemäß Beispiel 9a), suspendiert in 300 ml n-Butylacetat, wurden innerhalb von 5 Minuten hinzugegeben und 30 Minuten unter N2 bei 90°C nachgerührt.

Nach dem Abkühlen auf 30°C, wurden 900 ml Wasser unter Rühren hinzugefügt. 123 g Natriumhydroxid Lösung (32 %ig) wurden tropfenweise bei 35 bis 40°C hinzugefügt, um einen pH von 8,0 einzustellen. Das Reaktionsgemisch wurde auf Raumtemperatur abgekühlt und das Produkt abgesaugt und im Vakuum getrocknet.

Man erhielt in 88 %iger Ausbeute weiße Kristalle des n-Nonanoylamidocaproyloxybenzolsulfonsäure Natriumsalzes.

Gleiche Ergebnisse, d.h. Ausbeuten von mehr als 90 % an Acyloxybenzolsulfonat erhält man auch dann, wenn man bei der Trocknung des SPS das Restwasser bei 220°C während 30 Minuten entfernt. Dies zeigt, dass das erfindungsgemäße Verfahren bei der Trocknung des SPS sehr viel größere Variationsmöglichkeiten bezüglich Zeit und Temperatur zulässt als das Verfahren gemäß US 4,666,636. Dadurch ist das erfindungsgemäße Verfahren sehr viel besser im großtechnischen Maßstab ausführbar.

## Patentansprüche

1. Verfahren zur Herstellung von Acyloxybenzolsulfonaten durch Umsetzung von wasserfreien Phenolsulfonaten mit Carbonsäurederivaten, **dadurch gekennzeichnet, dass** man die Umsetzung mit einem Salz einer Phenolsulfonsäure durchführt, das einen Wassergehalt von weniger als 0,5 Gew.-% aufweist, und das mit einer Substanz mit basischen Eigenschaften in Kontakt gebracht wurde.

2. Verfahren nach Anspruch 1, **dadurch gekennzeichnet, dass** man die Umsetzung mit einem Salz einer Phenolsulfonsäure durchführt, das einen Wassergehalt von weniger als 0,2 Gew.-% aufweist.

3. Verfahren nach Anspruch 1, **dadurch gekennzeichnet, dass** man als Substanz mit basischen Eigenschaften solche Verbindungen einsetzt, die in wässriger Lösung unter Bildung von Hydroxylionen dissoziieren.

4. Verfahren nach Anspruch 1, **dadurch gekennzeichnet, dass** man als Substanz mit basischen Eigenschaften Alkali- oder Erdalkalioxide, -hydroxide, -carbonate, -hydrogencarbonate oder -phosphate einsetzt.

5. Verfahren nach Anspruch 1, **dadurch gekennzeichnet, dass** man als Substanz mit basischen Eigenschaften Natriumcarbonat, Natriumhydrogencarbonat und Natriumhydroxid oder die analogen Kaliumverbindungen einsetzt.

6. Verfahren nach Anspruch 1, **dadurch gekennzeichnet, dass** man 0,01 bis 10 Gew.-% an Substanz mit basischen Eigenschaften, bezogen auf Phenolsulfonat in Form des Dihydrats, einsetzt.

7. Verfahren nach Anspruch 1, **dadurch gekennzeichnet, dass** man die Substanz mit basischen Eigenschaften als Pulver, Slurry, Paste oder als wässrige Lösung einsetzt.

8. Verfahren nach Anspruch 1, **dadurch gekennzeichnet, dass** das Salz der Phenolsulfonsäure direkt nach dessen Isolierung mit der Substanz mit basischen Eigenschaften in Kontakt bringt.

9. Verfahren nach Anspruch 1, **dadurch gekennzeichnet, dass** man das Salz der Phenolsulfonsäure während dessen Trocknung mit der Substanz mit basischen Eigenschaften in Kontakt bringt.
